Europäisches Patentamt

European Patent Office

Office européen des brevets

(11) Publication number: **0 241 295 B1**

(12) EUROPEAN PATENT SPECIFICATION

(45) Date of publication of patent specification: **12.08.92**   (51) Int. Cl.⁵: **A61K 6/00**, A61K 31/00,
                                                                      A61K 31/78

(21) Application number: **87303114.0**

(22) Date of filing: **09.04.87**

(54) **Pharmaceutical composition for use in desensitizing hypersensitive dentin.**

(30) Priority: **10.04.86 JP 82839/86**

(43) Date of publication of application:
**14.10.87 Bulletin 87/42**

(45) Publication of the grant of the patent:
**12.08.92 Bulletin 92/33**

(84) Designated Contracting States:
**BE CH DE GB LI**

(56) References cited:
**FR-A- 2 187 826**

**CHEMICAL ABSTRACTS, vol. 82, no. 4, 27
January 1975, Columbus, OH (US); K.KOJIMA
et al., p. 33, no. 17554z**

(73) Proprietor: **Masuhara, Eiichi
2-5-10 Honkomagome
Bunkyo-ku Tokyo(JP)**

(72) Inventor: **Masuhara, Eiichi
2-5-10, Honkomagome
Bunkyo-ku Tokyo(JP)**
Inventor: **Hosoda, Hiroyasu
2-36-3 Hakusan
Bunkyo-ku Tokyo(JP)**

(74) Representative: **Woods, Geoffrey Corlett et al
J.A. KEMP & CO. 14 South Square Gray's Inn
London WC1R 5EU(GB)**

# Description

The present invention relates to a pharmaceutical composition comprising N-methacryloyl aminosalicylic acid which is capable of desensitizing hypersensitive dentin or eliminating odontalgia or toothache, and to the use of N-methacryloyl aminosalicylic acid in preparing the pharmaceutical composition.

If part of the enamel of a tooth is cracked or broken as a result of caries or trauma, the endodontium will be tremendously hypersensitive to an exgenic stimulus transmitted directly through the dentin. Accordingly, a patient suffering from such wound will often feel acute pain when the affected tooth is merely in contact with cold water or air. Particularly, a patient suffering from odontalgia at the neck of a tooth, where the enamel layer is very thin and therefore liable to be broken, would be sometimes not be able to eat or drink due to violent toothache.

It has now been found that N-methacryloyl aminosalicylic acid has an excellent desensitizing activity. The present invention therefore provides a pharmaceutical composition for use in desensitizing hypersensitive dentin or eliminating odontalgia, which composition comprises a N-methacryloyl aminosalicylic acid as active ingredient and a pharmaceutically acceptable carrier or diluent.

The odontalgia may be caused by, for example, exgenic stimulus, caries or erosion.

N-methacryloyl aminosalicylic acid, referred to hereinafter as NMSA, consists of an aminosalicylic acid moiety and a methacryl moiety linked to each other via an amide group. NMSA may be prepared in a conventional manner in which methacryl chloride is reacted with aminosalicylic acid followed by recrystallization in a mixed solvent of ethyl acetate and acetone. NMSA is sparingly soluble in water and has little toxicity. The preferred NMSA is N-methacryloyl 5-aminosalicylic acid (5-NMSA).

The pharmaceutically acceptable carrier or diluent may be any liquid carrier known in the art provided that an effective amount of NMSA may be dissolved therein. Such a carrier or diluent includes, for example, acetone, ethyl alcohol, ethyl acetate and methyl methacrylate, acetone being preferred. The NMSA is preferably present in the composition in an amount of from 0.5 to 20 %, more preferably from 1 to 15 %, most preferably from 2 to 10 %, by weight of the composition.

The desensitizing composition of the invention may be applied directly to exposed dentin in liquid form.

The composition may further comprise a pharmaceutically acceptable compound which has an unsaturated group and which is copolymerizable with the NMSA, and a polymerization initiator.

While the composition comprising NMSA and the carrier or diluent is rapidly and temporarily effective in the desensitization, the composition additionally comprising the unsaturated compound and the initiator prolongs the desensitizing effect by copolymerizing the active NMSA with the unsaturated compound.

The term "a pharmaceutically acceptable compound which has an unsaturated group" in the context of the present invention means any compound which is pharmaceutically acceptable and which can be copolymerized with NMSA. Preferred compounds are those having a methacryl group such as methacrylate, dimethacrylate, trimethacrylate, tetramethacrylate, and polymers thereof, generally having a molecular weight of from 10,000 to 1,000,000, and mixtures of two or more thereof. Methyl methacrylate, triethyleneglycol dimethacrylate and their polymers are more preferred. Some carriers or diluents, for example methyl methacrylate, can also serve as the compound which has an unsaturated group.

Any polymerization initiator used in known polymerization methods such as a redox system or photochemical sensitization system may be contained in the pharmaceutical composition, for example a peroxide, tertiary amine, tertiary boron, e.g. tri-n-butyl borane (TBBO), and camphor quinone.

The compound which has an unsaturated group is preferably present in the composition in an amount of from 0.1 to 99 %, more preferably from 1 to 15 %, most preferably from 2 to 10 %, by weight of the composition. The polymerization initiator may be contained in a suitable amount, generally from 0.01 to 1.0 % by weight of the pharmaceutical composition. Selection of the polymerization initiator can easily be made by those skilled in the art.

The pharmaceutical composition of this type should be stored until use in a cold or dark place in order to prevent copolymerization prior to application.

The pharmaceutical composition of the present invention in use is applied to the surface of exposed dentin, for example by impregnating a suitable medium such as a sponge or sanitary cotton with a suitable amount of the composition and then applying the medium to the dentin. A suitable dosage may be determined by those skilled in the art based on professional experience.

The NMSA in the pharmaceutical composition promptly interrupts the exgenic stimulus and advantageously demonstrates a remarkable desensitizing effect.

When the pharmaceutical composition comprising the compound which has an unsaturated group and the polymerization initiator is applied, the above desensitizing effect may be kept for a long time because NMSA can be retained on the surface of the applied dentin as a component of a membrane formed as a result of the copolymerization initiated by free radicals produced by irradiation of light or oxidation and reduction.

Furthermore, when a commercially available methyl methacrylate-type adhesive resin is pressed on the surface of the affected dentin after having been coating with the pharmaceutical composition according to the present invention, the resin will tightly adhere to the dentin via the NMSA with an adhesive strength of about 170 kg/cm$^2$ to give very quick relief of toothache.

Alternatively, after the pharmaceutical composition comprising the compound which has an unsaturated group and the polymerization initiator is applied and cured on the surface of the affected dentin, a polyfunctional methacrylate type composite resin, also commercially available, may be pressed and cured on the previously cured composition to obtain an adhesive strength of about 150 kg/cm$^2$; this eliminates the toothache at the endodontium.

As seen from the above, the pharmaceutical composition according to the present invention can show a rapid and continuous desensitizing effect merely by applying it on the surface of the exposed dentin. In addition, it may also serve as an effective adhesive which binds the dentin and an adhesive resin (synthetic resin) or repairing composite resin together. Thus, the pharmaceutical composition of the present invention provides a great progress in the dental therapy of caries.

The present invention also provides the use of NMSA in the preparation of a pharmaceutical composition for use in desensitizing hypersensitizing dentin or eliminating odontalgia.

The following Examples further illustrate the invention.

EXAMPLE 1

A piece of sponge (2x2x2 mm) was sufficiently impregnated with a pharmaceutical composition comprising 95 parts by weight of acetone and 2 parts by weight of 5-NMSA. When hypersensitive dentin due to endodontitis in an early stage had been coated with a thin layer of the above composition by means of the sponge, the odontalgia was instantly eliminated.

EXAMPLE 2

A pharmaceutical composition comprising 96 parts by weight of methyl methacrylate, 2 parts by weight of 5-NMSA and 0.01ml of tri-n-butyl borane (TBBO) as a polymerization initiator at ambient temperature was immediately absorbed into a small piece of sponge and applied to the surface of a prepared cavity formed in a vital tooth in an operation. The composition penetrated into the dental canaliculi and was copolymerized and cured a few minutes later to form a polymeric membrane on the surface of the cavity. By this treatment the toothache at the cavity was immediately mitigated. Furthermore the desensitizing activity of the applied composition remained for a long time as a result of the formation of the membrane, which could, on the other hand, also protect the endodontium.

EXAMPLE 3

A pharmaceutical composition according to the present invention in a form of slurry paste was prepared by adding 0.01ml of TBBO to a mixture (0.7ml) of 98 parts by weight of methyl methacrylate and 2 parts by weight of 5-NMSA followed by the incorporation of polymethyl methacrylate in a fine powder (1mg). The same treatment as in Example 2 was performed with this pharmaceutical composition to obtain the excellent desensitizing effect described in Example 2. The cured composition firmly adhered to the dentin with an adhesive strength of 170 kg/cm$^2$.

When a commercially available repairing composite resin was further pressed on the surface of the cured composition resin, both resins were tightly bound to each other through a chemical bond, providing the affected tooth with an aesthetic repair as well as the continuous desensitizing effect.

EXAMPLE 4

A pharmaceutical composition according to the present invention was prepared by mixing 60 parts by weight of methyl methacrylate, 37 parts by weight of triethylene glycol dimethacrylate, 2 parts by weight of 5-NMSA and one part by weight of camphor quinone as a photo-polymerization initiator (photochemical sensitization agent) at ambient temperature and stored in a vessel shielded from light. After application of this pharmaceutical composition by means of a small piece of sponge on the surface of exposed dentin caused by caries or erosion, the composition was irradiated with light at 400 to 600 nm for 40 seconds to cure it. The cured membrane of the pharmaceutical composition rapidly interrupted the exgenic stimulus and continuously mitigated the odontalgia.

When a photo-polymerization type resin paste was further pressed on the cured membrane and irradiated with visible light for 30 seconds, the resin paste was cured and bound very firmly to the cured membrane via a chemical bond. Thus an aesthetic repair was obtained as well as a continuous desensitizing effect on the odontalgia due to the exgenic stimulus towards endodontium.

## Claims

1. A pharmaceutical composition for use in desensitizing hypersensitive dentin or eliminating odontalgia, which composition comprises N-methacryloyl aminosalicylic acid as active ingredient and a pharmaceutically acceptable carrier or diluent.

2. A pharmaceutical composition according to claim 1, wherein the N-methacryloyl aminosalicylic acid is N-methacryloyl 5-aminosalicylic acid.

3. A pharmaceutical composition according to claim 1 or 2, wherein the carrier or diluent is selected from acetone, ethyl alcohol, ethyl acetate and methyl methacrylate.

4. A pharmaceutical composition according to any one of the preceding claims, further comprising a pharmaceutically acceptable compound which has an unsaturated group and which is copolymerizable with the N-methacryloyl aminosalicylic acid, and a polymerization initiator.

5. A pharmaceutical composition according to claim 4 wherein the said compound having an unsaturated group is a compound having a methacryl group.

6. A pharmaceutical composition according to claim 5, wherein the said compound having an unsaturated group is a methacrylate, dimethacrylate, trimethacrylate or tetramethacrylate, a polymer thereof or a mixture of two or more thereof.

7. A pharmaceutical composition according to claim 6, wherein the said compound having an unsaturated group is methyl methacrylate or a mixture thereof with polymethyl methacrylate or triethyleneglycol dimethacrylate.

8. A pharmaceutical composition according to any one of claims 4 to 7, wherein the polymerization initiator is tri-n-butyl borane or camphor quinone.

9. Use of N-methacryloyl aminosalicylic acid in the preparation of a pharmaceutical composition for use in desensitizing hypersensitive dentin or eliminating odontalgia.

## Revendications

1. Composition pharmaceutique destinée à l'emploi pour la désensibilisation de la dentine hypersensible ou pour la suppression d'odontalgies, caractérisée en ce qu'elle comprend de l'acide N-méthacryloylaminosalicylique à titre d'ingrédient actif et un diluant, véhicule ou excipient pharmaceutiquement acceptable.

2. Composition pharmaceutique suivant la revendication 1, caractérisée en ce que l'acide N-méthacryloylaminosalicylique est l'acide N-méthacryloyl-5 amino-salicylique.

3. Composition pharmaceutique suivant la revendication 1 ou 2, caractérisée en ce que l'on choisit le diluant, véhicule ou excipient parmi l'acétone, l'alcool éthylique, l'acétate d'éthyle et le méthacrylate de méthyle.

4. Composition pharmaceutique suivant l'une quelconque des revendications précédentes, caractérisée en ce qu' elle comprend complémentairement un composé pharmaceutiquement acceptable, qui possède un groupe insaturé et qui est copolymérisable avec l'acide N-méthacryloylaminosalicylique et un amorceur de polymérisation.

5. Composition pharmaceutique suivant la revendication 4, caractérisée en ce que le composé qui comporte un groupe insaturé est un composé comportant un radical méthacryle.

6. Composition pharmaceutique suivant la revendication 5, caractérisée en ce que le composé qui possède un groupe insaturé est un méthacrylate, diméthacrylate, triméthacrylate ou tétraméthacrylate, un polymère d'un tel composé ou un mélange de deux ou de plus de deux d'entre eux.

7. Composition pharmaceutique suivant la revendication 6, caractérisée en ce que le composé qui possède un groupe insaturé est le méthacrylate de méthyle, ou un mélange de ce dernier avec du polyméthacrylate de méthyle ou du diméthacrylate de triéthylèneglycol.

8. Composition pharmaceutique suivant l'une quelconque des revendications 4 à 7, caractérisée en ce que l'amorceur de polymérisation est le tri-n-butylborane ou de la campho-quinone.

9. Utilisation de l'acide N-méthacryloylaminosalicylique pour la préparation d'une composition pharmaceutique destinée à la désensibilisation de la dentine hypersensible ou à la suppression d'odontalgies.

**Patentansprüche**

1. Pharmazeutische Zusammensetzung zur Verwendung bei der Desensibilisierung von überempfindlichem Dentin oder zur Eliminierung von Zahnschmerz, wobei die Zusammensetzung eine N-Methacryloylaminosalicylsäure als aktiven Bestandteil und einen pharmazeutisch akzeptablen Träger oder Verdünnungsmittel enthält.

2. Pharmazeutische Zusammensetzung nach Anspruch 1, dadurch **gekennzeichnet,** dass die N-Methacryloylaminosalicylsäure N-Methacryloyl-5-aminosalicylsäure ist.

3. Pharmazeutische Zusammensetzung nach Anspruch 1 oder 2, dadurch **gekennzeichnet,** dass der Träger oder das Verdünnungsmittel aus Aceton, Ethylalkohol, Ethylacetat und Methylmethacrylat ausgewählt ist.

4. Pharmazeutische Zusammensetzung nach einem der vorhergehenden Ansprüche, dadurch **gekennzeichnet,** dass sie weiterhin eine pharmazeutisch akzeptable Verbindung, die eine ungesättigte Gruppe aufweist und die mit der N-Methacryloylaminosalicylsäure copolymerisierbar ist, und einen Polymerisationsinitiator enthält.

5. Pharmazeutische Zusammensetzung nach Anspruch 4, dadurch **gekennzeichnet,** dass die Verbindung mit einer ungesättigten Gruppe eine Verbindung mit einer Methacrylgruppe ist.

6. Pharmazeutische Zusammensetzung nach Anspruch 5, dadurch **gekennzeichnet,** dass die Verbindung mit einer ungesättigten Gruppe ein Methacrylat, Dimethacrylat, Trimethacrylat oder Tetramethacrylat, ein Polymer davon oder eine Mischung von zwei oder mehreren davon ist.

7. Pharmazeutische Zusammensetzung nach Anspruch 6, dadurch **gekennzeichnet,** dass die Verbindung mit einer ungesättigten Gruppe Methylmethacrylat oder eine Mischung davon mit Polymethylmethacrylat oder Triethylenglykoldimethacrylat ist.

8. Pharmazeutische Zusammensetzung nach einem der Ansprüche 4 bis 7, dadurch **gekennzeichnet,** dass der Polymerisationsinitiator Tri-n-butylboran oder Kampferchinon ist.

9. Verwendung von N-Methacryloylaminosalicylsäure für die Herstellung einer pharmazeutischen Zusammensetzung für die Verwendung zum Desensibilisieren von überempfindlichem Dentin oder zur Eliminierung von Zahnschmerz.